# EUROPEAN PATENT SPECIFICATION

(11) **EP 1 831 146 B1**
(45) Date of publication and mention of the grant of the patent: **21.05.2008**
(21) Application number: 05824612.5
(22) Date of filing: 21.12.2005
(51) Int. Cl.: C07C 51/14, C07C 67/38, C07C 271/22, C07C 63/331, C07C 63/06, C07C 69/82, C07C 63/70, C07C 65/32, C07C 65/21, C07C 63/26, C07C 61/29

(54) **HYDROXYCARBONYLATION OF ARYL AND VINYL BROMIDES**
HYDROXYCARBONYLIERUNG VON ARYL- UND VINYLBROMID
HYDROXYCARBONYLATION DE BROMURES D'ARYLE ET DE VINYLE

(30) Priority: 22.12.2004 DE 102004063022
(43) Date of publication of application: 12.09.2007
(73) Proprietor: Finorga, 38670 Chasse sur Rhône (FR)
(72) Inventor: LEMEUNE, Alla, F-69006 Lyon (FR); MIGNONAC, Sylviane, F-69007 Lyon (FR); BERGER, Philippe, F-38440 St Jean de Bournay (FR); CAILLE, Jean-Claude, F-49000 Angers (FR)
(74) Representative: Hirsch & Associés
(86) International application number: PCT/EP2005/014211
(87) International publication number: WO 2006/066975

(56) References cited:
- CACCHI, S. ET AL: "Palladium-Catalyzed Hydroxycarbonylation of Aryl and Vinyl Halides or Triflates by Acetic Anhydride and Formate Anions" ORGANIC LETTERS, vol. 5, no. 23, 2003, pages 4269-4272, XP002371991 cited in the application
- CACCHI, SANDO ET AL: "Palladium -catalyzed hydroxycarbonylation of vinyl and aryl triflates: synthesis of .alpha.,.beta.-unsaturated and aromatic carboxylic acids" TETRAHEDRON LETTERS , 33(27), 3939-42 CODEN: TELEAY; ISSN: 0040-4039, 1992, XP002371992 cited in the application

## Description

The object of the present invention is the transition metal-catalysed hydroxycarbonylation of aryl and vinyl bromides.

The transition metal-catalysed carbonylation of different organic substrates is of major significance in industrial organic synthesis [(a) Conquhoun, H. M. et al., Carbonylation, Direct Synthesis of Carbonyl Compounds, Plenum Press, New York, (1991). (b) Tsuji, J., Palladium Reagents and Catalysts, Wiley : Chichester, (1995). (c) Beller, M., Applied Homogeneous Catalysis with Organometallic Compounds 1; Comils, B.; Hemnann, W. A., Eds., VCH: Weinheim, (1996). (d) Grushin, V. V.; Alper, H., Chem. Rev., 94, 1047, (1994)]. The hydroxycarbonylation of aryl and vinyl halogenides or triflates has drawn considerable attention [(a) Cacchi, S et al.., G., Tetrahedron Lett., 26, 1109, (1985). (b) Pri-Bar, L; Buchman, O., J. Org. Chem., 53, 624, (1988). (c) Cacchi, S.; Lupi, A., Tetrahedron Lett., 33, 3939 (1992). (d) Grushin, V. V.; Alper, H., J. Am. Chem. Soc., 117, 4305 (1995). (e) Ziolkowski, J. J.; Trzeciak, A. M., J. Mol. Cat. A: Chem., 154, 93 (2000). (g) Calo, V. et al., J. Organomet. Chem., 645, 152 (2002)].. Usually, carbon monoxide is used as a substrate for such reactions. This inexpensive technical gas, readily available in industrial quantities, is highly toxic and must be stored in stainless steel containers [(3) The Merck Index: An Encyclopedia of Chemicals, Drugs and Biologicals; Whitehouse Station, NJ, (2001). The use of carbon monoxide in chemical plants requires special equipping and expensive safety measures. It is accordingly highly desirable to develop a new and secure technology, by which carbon monoxide does not have to be introduced directly to reaction mixtures, rather is produced *in situ*. In addition one particularly high interest in such technology is the use in the pharmaceutical industry. The wide use of automated combinatorial chemistry for the discovery of new drugs requires the replacement of gaseous substrates by liquid and solid materials, which are easier to handle. Also, the production of a plurality of substances on the kilogram scale for pre-clinical studies is often limited, since the medium-scale pharmaceutical industry is not in a position to work safely with carbon monoxide.

In recent years a few sources has been reported for carbon monoxide. Carbonylation was achieved using reagents, which contained a carbonyl group such as for example methyl formate [(a) Lee, J. S. et al., Appl. Catal., 57, 1 (1990). (b) Carpentier, J.-F. et al., Tetrahedron Lett, , 32, 4705 (1991)]. Alper and Grushin, Organometallics, 12, 3846 (1993) have described the catalytic hydroxycarbonylation of aryl iodide, vinyl and benzyl halides by carbon monoxide produced *in situ* from chloroform and aqueous alkalis and, more recently, Cacchi et al., Org. Lett., 5, 4269 (2003) have reported on the use of acetic acid anhydride and lithium formate as starting materials for carbon monoxide. A mixed anhydride of acetic acid and formic acid was postulated as carbon monoxide source under these conditions. Aryl iodides were hydroxycarbonylated in this way under mild conditions. Aryl bromides, the more interesting substrates for industrial applications, reacted however only sluggishly under the abovementioned conditions. In some cases PdCl₂(dppp) proved to be an efficacious pre-catalyst, though reaction ran extremely slowly (64 hours) and resulted in only moderate yields of the desired products. The study of Pd-catalysed hydroxycarbonylation of aryl bromides using the carbon monoxide source described by Cacchi et al. 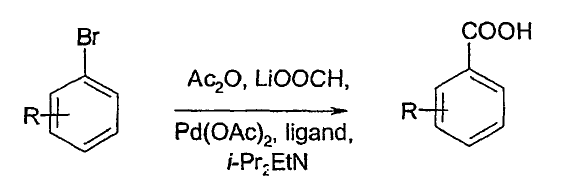 through the hydroxycarbonylation of *p*-bromobiphenyl was disappointing. As put forward by Cacchi et al., 2 equivalents of lithium formate, 3 equivalents of acetic acid anhydride and 2 equivalents of *i*-Pr₂EtN (diisopropyl ethyl amine) in DMF (dimethylformamide) were mixed with one another at room temperature for 1 hour, after which *p*-bromobiphenyl, lithium chloride and the Pd catalyst were added, and reaction was carried out at 80°C. Even the simple substitution of the specified PdCl₂/dppp (1,3-bis[diphenylphosphino]propane)dichloropalladium for a better available catalytic system such as Pd(OAc)₂/dppp and PdCl₂/dppp produced the desired acid only in a low yield (Table 1, No. 1, 2).

All methods described to date for hydroxycarbonylation of aryl and vinyl bromides are therefore to be considered as not particularly satisfactory.

The object of the present invention was therefore to overcome the drawbacks of the prior art.

According to the invention, the problem was solved by a novel and efficient catalytic system for the hydroxycarbonylation reaction of aryl and vinyl bromides.

It has now been evidenced that the preparation of aryl or vinyl carboxylic acids via Pd-catalysed hydroxycarbonylation of aryl and vinyl bromides, reacting the aryl or vinyl bromides with a carbon monoxide source comprising lithium formate and a carboxylic acid anhydride (for example acetic acid anhydride), in the presence of the combination of palladium complexes with the chelating ligand dppf(1,1-bis[diphenylphosphino]ferrocene) produced the desired product in a good yield.

According to the invention the Pd catalyst can be chosen from the combinations of palladium complexes (pre-catalyst) : PdCl₂ (dichloropalladium), Pd₂(dba)₃ (dba = dibenzylideneacetone) or Pd(OAc)₂ diacetoxypalladium with the chelating ligand dppf.

According to the invention the reaction is advantageously carried out in the presence of a tertiary amine such as triethylamine, diisopropyl ethyl amine, tetramethylethylenediamine, tributylamine, pyridine, N-methylmorpholine, tetramethylurea, methylpyrrolidinone, 4-dimethylaminopyridine, proton sponge or dimethylaniline, in a polar solvent such as an amide (dimethylformamide, N-methyl-2-pyrrolidone for example) at a temperature from 60 to 200°C, preferably at a temperature from 80 to 150°C and more preferably at a temperature from 80 to 120°C, the temperature of 120°C being the more preferred. Optionally, the reaction can be carried out in the presence of lithium chloride.

According to the invention, the molar amount of the chelating ligand is from 1 to 20%, preferably 1 to 10%, and the molar amount of the pre-catalyst Pd complex is comprised from 1 to 5%. The ratio pre-catalyst / ligand is advantageously comprised from 0.25 to 1, preferably from 0.3 to 1 and more preferably about 1. The ratio pre-catalyst / ligand of 1 being the more preferred one.

Among aryl and vinyl bromides, the reaction applies to a variety of substrates including a substantial range, and based on neutral aryl bromides, electron-poor and electron-rich aryl bromides which produces the desired acids with good yields. The variety of substrates includes also dibromo-substituted substrates. This shows that the inventive hydroxycarbonylation can surprisingly also be used for the synthesis of dicarboxylic acids. The inventive hydroxycarbonylation can further be used surprisingly for the conversion of vinyl bromides in the corresponding acids with high yields : as a non-limitative example, 2-bromoindene produced the corresponding acid.

According to the invention, the aryl bromides can be chosen from the bromides where the aryl is a substituted or unubstituted 5 to 7 members single aromatic or heteroaromatic radical comprising 0 to 4 heteroatoms chosen from oxygen, sulphur or nitrogen or a bi or tricyclic substituted or unsubstituted aromatic or heteroaromatic radical comprising 6 to 14 members and 0 to 4 heteroatoms chosen from oxygen, sulphur or nitrogen and which can be saturated or partially saturated on the cycles that are not bromo-substituted. The substituants can be advantageously chosen from halogen atoms, or radicals such as alkyl, halogenoalkyl or polyhalogenoalkyl (trifluoromethyl for example), alkoxy, acyl, alkoxycarbonyl, alkylaminoalkyl, dialkylaminoalkyl, alkoxycarbonylaminoalkyl or phenyl, the alkyl portions or radicals and the alkyl portion in the acyl radical being linear or branched and having 1 to 10 carbon atoms.

More preferred are the aryl radicals chosen from phenyl or naphtyl, that can be substituted with halogen atoms chosen from chloro, fluoro, bromo or iodo, or substituted with radicals chosen from linear or branched alkyl or alkoxy having 1 to 4 carbon atoms, trifluoromethyl, acyl or alkoxycarbonyl where the alkyl portions are linear or branched and contain 1 to 4 carbon atoms, dialkylaminoalkyl, methoxycarbonylaminoalkyl, ethoxycarbonylaminoalkyl or t.butoxycarbonyl-aminoalkyl or phenyl.

According to the invention, the vinyl bromides can be chosen from the substituted or unsubstituted bromides where the vinyl radical is a vinyl derivative where the bromo is substituted on an unsaturated carbon and where the radical can be an aliphatic linear or branched radical having from 2 to 10 carbon atoms and that can comprise heteroatoms chosen from oxygen, sulphur or nitrogen or a mono, bi or tri cyclic radical comprising from 5 to 14 members, that can be carbocyclic or heterocyclic comprising 0 to 4 heteroatoms chosen from oxygen, sulphur or nitrogen, and which can be partially saturated and where the bromo is substituted on an unsaturated carbon. The substituants can be chosen from the substituants mentioned above for the aryl bromide.

More preferred are the vinyl radical chosen from vinyl or indene-2-yl that can be substituted with substituants chosen from the substituants mentioned above for the aryl bromide.

According to the invention, the concentration of the vinyl or aryl bromide is advantageously from 0.01 to 1 M, preferably from 0.1 to 0.8 M and more preferably from 0.1 to 0.4 M. The reaction is preferably carried out on diluted solutions of the starting vinyl or aryl bromide.

According to the invention the more preferred examples are the processes where :
- *N*-*tert*-butoxycarbonyl-*N*-methyl-4-bromobenzylamine is reacted with a carbon monoxide source comprising lithium formate and acetic acid anhydride, in the presence of diisopropyl ethyl amine and in the presence of the combination of palladium complexe Pd₂(dba)₃ with the chelating ligand dppf (1,1-bis[diphenylphosphino]ferrocene) in the ratio palladium pre-catalyst/ligand of 5/20, at a temperature of 120°C, in dimethylformamide, in the presence of lithium chloride ;
- *N-tert*-butoxycarbonyl-*N*-methyl-4-bromobenzylamine is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 5/5, at a temperature of 120°C, in N-methyl-pyrrolidone ;
- *N-tert*-butoxycarbonyl-*N*-methyl-4-bromobenzylamine is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 2.5/2.5, at a temperature of 120°C, in dimethylformamide ;
- bromotoluene is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 1/1 at a temperature of 120°C in dimethylformamide ;
- 4-bromofluorobenzol is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenyl-phosphino]ferrocene) in the ratio palladium pre-catalyst/ligand of 1/1 at a temperature of 120°C in dimethylformamide ;
- 4-anisole bromide is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenyl-phosphino]ferrocene) in the ratio palladium pre-catalyst/ligand of 1/1 at a temperature of 120°C in dimethylformamide ;
- 2-bromoindene is reacted with a carbon monoxide source comprising lithium formate and acetic acid anhydride, in the presence of diisopropyl ethyl amine and in the presence of the combination of palladium complexe Pd(OAc)₂ with the chelating ligand dppf (1,1-bis[diphenylphosphino]ferrocene) in the ratio palladium pre-catalyst/ligand of 1/1 at a temperature of 120°C in dimethylformamide ;
- 1,4-dibromobenzol is reacted with a carbon monoxide source comprising lithium formate and acetic acid anhydride, in the presence of diisopropyl ethyl amine and in the presence of the combination of palladium complexe Pd(OAc)₂ with the chelating ligand dppf (1,1-bis[diphenyl-phosphino]ferrocene) in the ratio palladium pre-catalyst/ligand of 1/1 at a temperature of 120°C in dimethylformamide ;

The following examples illustrate the invention.

### EXAMPLES

### Example 1

### Evaluation of ligands for aryl bromides hydroxycarbonylation

With ligand screening of a series of phosphine ligands using *p*-bromobiphenyl as model substrate (Table 1) only dppf (assay No. 9) give the desired compound in a high yield, while P(*t*-Bu)₃, P(*o*-tol)₃, PPh₃ and BINAP [2,2'-bis(diphenylphosphino)-1,1'-binaphtyl] were less efficient or ineffective. Reaction conditions : 2 mmol 4-bromobiphenyl, 2 equiv. acetic acid anhydride, 3 equiv. lithium formate, 2 equiv. *i-*Pr₂EtN, 3 equiv. LiCl in 9 ml DMF were caused to react with one another for 24 hours at 80°C. After cooling the reaction mixture was diluted with ethyl acetate, washed with 2 N HCl, dried over Na₂SO₄ and concentrated under reduced pressure. The residue was analysed with ¹H and ¹³C NMR. 1,1'-bis(di-*iso*propylphosphine)ferrocene (-)-(R)-1-[(S)-2-(diphenylphosphine)ferrocenyl]ethyl-methylether (R)-(-)-1-[(S)-2-(dicyclohexylphosphine)ferrocenyl]ethyldicyclohexylphosphine

**Table 1**

| **Assay No.** | **Pd pre-catalyst (mol %)** | **Ligand mol%** | **NMR yield %** |
|---|---|---|---|
| Comparative assays | | | |
| 1 | Pd₂(dba) (5) | dppp* (10) | 0 |
| 2 | PdCl₂ (10) | dppp (10) | 0 |
| 3 | Pd₂(dba)₃ (5) | P(*t*-Bu)₃ (10) | 10 |
| 4 | Pd₂(dba)₃ (5) | P(*t*-Bu)₃ (20) | 5 |
| 5 | Pd₂(dba)₃ (5) | P(*o*-tol)₃ ** (30) | 10 |
| 6 | Pd₂(dba)₃ (5) | P(*o*-tol)₃ (60) | 5 |
| 7 | Pd₂(dba)₃ (5) | P(Ph)₃ (30) | 0 |
| 8 | Pd₂(dba)₃ (5) | BINAP (20) | 25 |

| Assay according to the invention | | | |
|---|---|---|---|
| 9 | Pd₂(dba)₃ (5) | dppf (20) | 70 |

| | | | |
|---|---|---|---|
| * dppp : 1,3-bis(diphenylphosphino)propane ** o-tol : o-tolyl | | | |

### Example 2

### Optimisation of the hydroxycarbonylation temperature of reaction, palladium source, ratio precatalyst / ligand and optional amount of LiCl additive

Under the conditions of example 1, *N*-*tert*-butoxycarbonyl-*N*-methyl-4-bromobenzylamine was converted into the desired acid. The results are shown in Table 2. 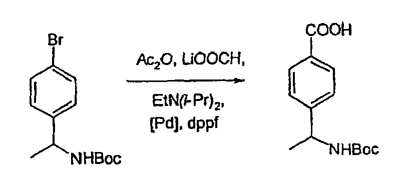 Reaction conditions : 1 (or 2) mmol bromide, 2 equivalents acetic acid anhydride, 3 equivalents lithium formate, 2 equivalents *i*-Pr₂EtN, in 5 (or 10) ml DMF. ^{b} The reaction was conducted in the presence of 3 equiv. LiCl. ^{c} NMR yield. After cooling the reaction mixture was diluted with ethyl acetate, washed with 2 N HCl, dried over Na₂SO₄ and concentrated under reduced pressure. The residue was analysed via ¹H and ¹³C NMR. ^{d} HPLC yields. ^{e} Isolated yield. ^{f} NMP (N-methyl pyrrolidone) was used as solvent. ^{g} DMSO (dimethylsulfoxyde) was used as solvent.

**Table 2**

| **Assay No.** | **Pd pre-catalyst (mol%)** | **Ligand mol%** | **Temp. °C** | **Time h** | **Yield %** |
|---|---|---|---|---|---|
| 1^{b} | Pd₂(dba)₃ (5) | 20 | 80 | 20 | 35^{c} |
| 2 | Pd₂(dba)₃ (5) | 20 | 80 | 20 | 45^{c} |
| 3^{b} | Pd₂(dba)₃ (5) | 20 | 60 | 20 | 15^{c} |
| 4^{b} | Pd₂(dba)₃ (5) | 20 | 120 | 2 3 | 83^{d} 86^{d} (70)^{e} |
| 5^{b} | Pd₂(dba)₃ (2.5) | 10 | 120 | 5 | 61^{d} |
| 6 | Pd₂(dba)₃ (2.5) | 10 | 120 | 5 | 81^{d} |
| 7 | PdCl₂ (5) | 10 | 120 | 2.5 | 77^{d} |
| 8 | Pd(OAc)₂ (5) | 10 | 120 | 2.5 | 81^{d} |
| 9 | Pd(OAc)₂ (5) | 5 | 120 | 2.5 | 93^{d} |
| 10 | Pd(OAc)₂ (5) | 15 | 120 | 2.5 | 86^{d} |
| 11^{f} | Pd(OAc)₂ (5) | 5 | 120 | 2.5 | 96^{d} |
| 12^{g} | Pd(OAc)₂ (5) | 5 | 120 | 2.5 | 64^{d} |
| 13 | Pd(OAc)₂ (2.5) | 2.5 | 120 | 2.5 | 97^{d} |
| 14 | Pd(OAc)₂ (1) | 1 | 120 | 2.5 | 92^{d} |

The assays indicated that the temperature acts on the yield of the hydroxycarbonylation reaction, since both the oxidative addition of a bromide and the thermal decomposition of the intermediate acetic acid / formic acid anhydride is determined by this factor.

An improvement was achieved by a rise in temperature (Table 2, assays No. 1, 3, 4). When the reaction was carried out at 120°C, this resulted in a significant rise in the yield. In comparison to the details disclosed by Cacchi et al. no favourable effect of LiCl was observed, when dppf was used as ligand at 80°C or 120°C (Table 2, assays No. 2 and 6 compared to 1 and 5). A change in the palladium source yielded only a moderate effect, Pd₂(dba)₃, Pd(OAc)₂ and PdCl₂ were effective pre-catalysts (Table 2, assays No. 6 to 8). If the pre-catalyst / ligands ratio was changed, slightly better yields resulted from using an equimolar ratio of Pd(OAc)₂ and dppf (Table 2, assays No. 8 to 10). The reaction ran well in polar solvents such as DMF and NMP (Table 2, No. 9, 11).

### Example 3

### Examination of the substrate concentration for the hydroxycarbonylation of bromide : N-tert-butoxycarbonyl-N-methyl-4-bromobenzplamine.

Reaction conditions : 0.01-0.75 mol bromide, 2 equiv. acetic acid anhydride, 3 equiv. lithium formate, 2 equiv. base, 1 mol % Pd(OAc)₂, 1 mol % dppf

The product yield varies with the aryl bromide concentration. The hydroxycarbonylation reaction proceeded smoothly in diluted solutions of aryl bromide in DMF (Table 3).

**Table 3**

| **Assay No.** | **[ArBr] M** | **Isolated** |
|---|---|---|
| 1 | 0.18 | 75 |
| 2 | 0.22 | 79 |
| 3 | 0.66 | 44 |
| 4 | 0.80 | 38 |

### Example 4

### Application of hydroxycarbonylation reaction to different vinyl and aryl bromides

To understand the limits of this reaction, a series of bromides was examined. The reaction conditions used in examining different aryl bromides were : aryl bromide (1 equivalent, 0.18 M), Pd(OAc)₂ / dppf (3-5 mol %), *i*-Pr₂EtN (2 equivalents), LiO₂CH (3 equivalents), Ac₂O (2 equivalents) in DMF at 120°C. The results are specified in Table 4. The amount of the catalytic system was not optimized, and instead 3-5 mol-% of catalytic system were added to ensure complete conversion of the starting bromide.
Reaction conditions : 2 mmol bromide, 2 equiv. acetic acid anhydride, 3 equiv. lithium formate, 2 equiv. *i*-Pr₂EtN, Pd(OAc)₂: dppf ratio 1:1 in 9 ml DMF. ^{b} 33 % in the presence of 1 mol% Pd(OAc)₂ ^{c} 36 % in the presence of 3 mol% Pd(OAc)₂ ^{d} 20% benzoic acid was obtained.

| **Assay No.** | **Substrate** | **Pd(Oac)₂ mol%** | **Time, h** | **Isolated yield %** |
|---|---|---|---|---|
| 1 | 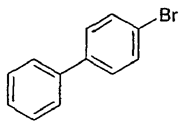 | 3 | 5 | 74^{b} |
| 2 | 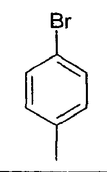 | 5 | 2 | 89 |
| 3 | 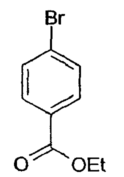 | 5 | 1 | 79 |
| 4 | 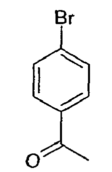 | 5 | 1 | 71 |
| 5 | 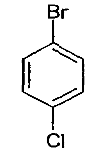 | 5 | 3 | 76 |
| 6 | 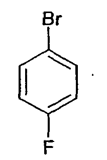 | 5 | 1 | 82 |
| 7 | 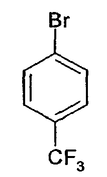 | 5 | 1 | 74 |
| 8 | 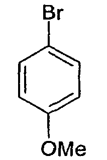 | 5 | 5 | 82 |
| 9 | 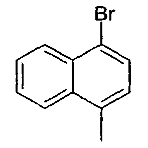 | 5 | 4 | 61^{c} |
| 10 | 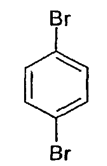 | 5 | 2 | 75^{d} |
| 11 | 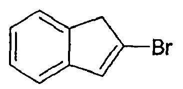 | 5 | 1 | 83^{c} |

### Table 4

Surprisingly, the hydroxycarbonylation reaction according to the invention can be applied to a substantial range of substrates and starting from neutral aryl bromides, electron-poor and electron-rich aryl bromides, produces the desired acids with good yields (Table 4, assays No. 1 to 8). 4-(trifluormethyl)-benzoic acid, which now has a series of industrial applications, can be produced for example with a yield of 74% (Table 4, Assay No. 7). Tolerance relative to different functional groups such as keto, ester or ether groups is of particular advantage under these conditions. Bromonaphthalenes can be likewise converted with good yields in the corresponding acids (Table 4, Assay No. 9). The inventive hydroxycarbonylation reaction can surprisingly also be used for the synthesis of dicarboxylic acids. Under the inventive conditions terephthalic acid can be obtained from 1,4-dibromobenzol with a yield of 75% (together with 20% benzoic acid) (Table 4, Assay No. 10). The inventive hydroxycarbonylation can further be employed surprisingly for the conversion of vinyl bromides in the corresponding acids. 2-bromoindene produced the corresponding acid with a yield of 80% under standard conditions (Table 4, Assay No. 11).

### Example 5

### General experimental procedure.

A solution of HCOOLi (312 mg, 6 mmol), *i*-Pr ₂NEt (697 *µ*l, 4 mmol), acetic acid anhydride (377 *µ*l, 4 mmol) in water-free DMF (9 ml) was stirred at room temperature for 1 hour in an inert atmosphere. Then aryl bromide (2 mmol), Pd(OAc)₂ (22.5 mg, 0.10 mmol) and dppf (55.4 mg, 0.10 mmol) were added. The reaction mixture was stirred at 120°C until the bromide had reacted fully (via HPLC). After cooling, 20 ml ethyl acetate and 20 ml water were added and the reaction mixture was adjusted with concentrated HCl, to pH 1. Two phases were separated and the aqueous layer was extracted with ethyl acetate (2 x 10 ml). The pure acid was isolated through extraction of the combined organic phase, with aqueous sodium hydroxide (10%) (3 x 10 ml), acidification of the aqueous phase to pH 1 with concentrated HCl, and then repeated extraction with ethyl acetate (3 x 15 ml). The combined organic layers were dried over Na₂SO₄ and concentrated under reduced pressure at 80°C. In a few cases (Table 4, Assays No. 3, 4, 11) the pure acids were isolated by chromatography (SiO₂; n-heptane/ethyl acetate 50/50). The ¹H- and ¹³C NMR spectra of the isolated acids (Table 4) matched those mentioned in the literature [Aldrich Chemical spectra, FT-NMR,1].

*N*-(*tert*-butoxycarbonyl)-methylbenzylamine-4-carboxyl acid (Table 3, Assay No. 2) was obtained from 3.00 g (10 mmol) *N-tert*-butoxycarbonyl-*N*-methyl-4-brombenzylamine under the abovementioned conditions.

Mp 146 - 147°C

¹H NMR (CDCl₃) : δ = 1.28 (t, J = 7.9 Hz, 3 H), 1.35 (s, 9H), 4.56 (q, J = 7.9 Hz, 1H), 7.39 (d, J = 7.8 Hz, 2H), 7.89 (d, J = 7.8 Hz, 2H), 10.5 (br s, 1H).

¹³C NMR (CDCl₃): δ = 23.09, 28.70, 50.00, 78.30, 126.39, 129.56, 129.84, 151.10, 155.29, 167.68.

4-biphenylcarboxyl acid (Table 4, Assay No. 1) was obtained from 466 mg (2 mmol) 4-brombiphenyl under the abovementioned conditions.

Mp 224 - 225°C; lit. Mp (Aldrich catalog) 225 - 226°C.

¹H NMR (DMSO-*d6*): δ = 7.45 (m, 3 H), 7.71 (d, J = 6.6 Hz, 2H), 7.78 (d, J = 8.2 Hz, 2H), 8.01 (d, J = 8.2 Hz, 2H), 11.5 (br s, 1H).

¹³C NMR : δ = 127.76, 127.90, 129.23, 130.02, 130.57, 130.92, 139.97, 145.25, 168.08.

*p*-toluic acid (Table 4, Assay No 2) was obtained from 855 mg (5 mmol) 4-bromotoluene under the abovementioned conditions.

Mp 180 - 181 °C; lit. Mp (Aldrich catalog) 180 -182°C

¹H NMR (DMSO-*d6*): δ = 2.18 (s, 3 H), 7.25 (d, J = 8.1 Hz, 2H), 7.82 (d, J = 8.1 Hz, 2H), 11.5 (br s, 1 H)

¹³C NMR (DMSO-*d6*): δ=22.37, 129.93, 131.37, 132.36, 138.80, 168.85.

Terephthalic acid, monoethyl ester (Table 4, Assay No. 3) was obtained from 2.29 g (10 mmol) ethyl-4-bromobenzoate under the abovementioned conditions.

¹H NMR (DMSO-*d6*): δ = 1.32 (t, J = 7.9 Hz, 3 H), 4.32 (q, J = 7.9 Hz, 2H), 8.03 (br s, 4H), 11.5 (br s, 1H).

¹³C NMR (DMSO-*d6*): δ = 19.83, 66.90, 135.02, 135.17, 139.10, 140.60, 170.84, 172.32.

4-acetylbenzoic acid (Table 4, Assay No. 4) was obtained from 796 mg (4 mmol) 4-bromoacetophenone under the abovementioned conditions.

Mp 208 - 209°C; lit. Mp (Aldrich catalog) 208 - 210°C.

¹H NMR (DMSO-*d6*): δ = 2.60 (s, 3 H), 8.02 (s, 4H), 13.2 (br s, 1H).

¹³C N MR (DMSO-*d6*): δ = 28.15, 129.47, 130.71, 135.71, 141.04, 167.81, 198.87.

4-chlorobenzoic acid (Table 4, Assay No. 5) was obtained from 383 mg (2 mmol) 4-bromochlorbenzol under the abovementioned conditions.

Mp 238 - 240°C; lit. Mp (Aldrich catalog) 239 - 241°C.

¹H NMR (DMSO-*d6*): δ = 7.54 (d, J = 8.4 Hz, 2H), 7.92 (d, J = 8.4 Hz, 2H), 7.93 (br s, 1 H).

¹³C NMR (DMSO-*d6*): δ = 129.37, 130.38, 130.65, 144.29, 168.66.

4-fluorobenzoic acid (Table 4, Assay No. 6) was obtained from 383 mg (2 mmol) 4-bromofluorobenzol under the abovementioned conditions.

Mp 183 - 184°C; lit. Mp (Aldrich catalog) 182 - 184°C.

¹H NMR (DMSO-*d6*): δ = 7.54 (d, J = 8.4 Hz, 2H), 7.92 (d, J = 8.4 Hz, 2H), 7.93 (br s, 1 H).

¹³C NMR (DMSO-d6): δ = 116.95 (d, J = 22.6 Hz), 129.10, 133.65 (d, J = 10.3 Hz), 166.65 (d, J = 250.6 Hz), 168.14.

4-(trifluoromethyl)-benzoic acid (Table 4, Assay No. 7) was obtained from 383 mg (2 mmol) 4-bromochlorbenzol under the abovementioned conditions.

Mp 218 - 220°C; lit. Mp (Aldrich catalog) 219 - 220°C.

¹H NMR (DMSO-*d6*): δ = 7.85 (d, J = 8.2 Hz, 2H), 8.12 (d, J = 8.2 Hz, 2H), 12.3 (br s, 1 H).

¹³C NMR (DMSO-*d6*): δ = 124.97 (q, J = 270.5 Hz), 126.69 (q, J = 3.2 Hz), 131.25, 133.34(q, J = 31.5 Hz), 135.82, 167.35.

*p*-anisic acid (Table 4, Assay No. 8) was obtained from 718 mg (4 mmol) 4-anisole bromide under the abovementioned conditions.

Mp 183 - 184°C; lit. Mp (Aldrich catalog) 182 - 185°C.

¹H NMR (DMSO-*d6*): δ = 3.81 (s, 3H), 7.01 (d, J = 8.1 Hz, 2H), 7.88 (d, J = 8.1 Hz, 2H), 12.61 (br s, 1 H).

¹³C NMR (DMSO-*d6*): δ = 56.60, 114.98, 124.17, 132.50, 164.025, 168.15.

4-methyl-1-naphthoic acid (Table 4, Assay No. 9) was obtained from 442 mg (2 mmol) 1-bromo-4-methylnaphthalene.

Mp 179 - 181°C; lit. Mp (Aldrich catalog) 179 - 180°C.

¹H NMR (DMSO-*d6*): δ = 2.71 (s, 3 H), 7.45 (d, J = 8.1 Hz, 1 H), 7.65 (m, 2H), 8.06 (d, J = 8.1 Hz, 1 H), 8.09 (m, 1 H), 8.94 (m, 1 H), 12.9 (br s, 1 H).

¹³C NMR (DMSO-*d6*): δ = 22.82, 125.85, 126.88, 127.31, 128.38, 131.05, 132.17, 133.63, 140.98, 170.01.

Terephthalic acid (Table 4, Assay No. 10) was obtained from 472 mg (2 mmol) 1,4-dibromobenzol under the abovementioned conditions together with benzoic acid (20%).

Mp >300°C; lit. Mp (Aldrich catalog) >300°C.

¹H NMR (DMSO-*d6*): δ = 8.15 (s, 4 H), 13.41 (s, 2H).

¹³C NMR (DMSO-*d6*): δ =130.76, 135.93, 167.15.

Indene-2-carboxylic acid (Table 4, Assay No. 11) was obtained from 780 mg (4 mmol) 2-bromoindene under the abovementioned conditions.

¹H NMR (DMSO-*d6*): δ = 3.62 (d, J = 1.6 Hz, 2H), 7.32 (m, 2H), 7.53 (m, 2H), 7.68 (m, 1 H), 12.9 (br s, 1 H).

¹³C NMR (DMSO-*d6*): δ = 39.06, 122.85, 123.88, 126.49, 126.83, 137.95, 139.69, 142.12, 144.18, 165.23.

Various ligands containing a free ferrocene fragment were tested for hydroxycarbonylation of 4-bromobiphenyl. 1,1'-bis(di-isopropylphosphine)-ferrocene proved efficient. On the other hand low yields of acid in the presence of ferrocenes were obtained, which contained an attached heteroatom group such as (-)-(R)-1-[(S)-2-(diphenylphosphine)ferroceneyl]-ethylmethylether or (R)-(-)-1-[(S)-2-(dicyclohexylphosphine)ferroceneyl]-ethyldicyclohexylphosphine.

## Claims

1. A process for the preparation of aryl or vinyl carboxylic acids via Pd-catalysed hydroxycarbonylation of aryl and vinyl bromides, wherein the aryl or vinyl bromides are reacted with a carbon monoxide source comprising lithium formate and a carboxylic acid anhydride, in the presence of the combination of palladium complexes with the chelating ligand dppf (1,1-bis[diphenylphosphino]ferrocene).

2. A process according to claim 1, wherein the Pd catalyst is chosen from the combinations of palladium complexes (pre-catalyst) : PdCl₂, Pd₂(dba)₃ or Pd(OAc)₂ with the chelating ligand dppf.

3. A process according to claim 1 or 2, wherein the carboxylic acid anhydride is acetic acid anhydride.

4. A process according to any of claims 1 to 3, wherein the reaction is carried out in the presence of a tertiary amine, in a polar solvent at a temperature comprised from 60 to 200°C.

5. A process according to claim 4, wherein the tertiary amine is chosen from triethylamine, diisopropyl ethyl amine, tetramethylethylenediamine, tributylamine, pyridine, N-methylmorpholine, tetramethylurea, methylpyrrolidinone, 4-dimethylaminopyridine, proton sponge or dimethylaniline.

6. A process according to claim 4 or 5, wherein the reaction is carried out at a temperature from 80 to 150°C, preferably 80 to 120°C and more preferably 120°C.

7. A process according to any of claims 4 to 6, wherein the polar solvent is an amide.

8. A process according to claim 7, wherein the amide is chosen from dimethylformamide and N-methyl-2-pyrrolidone.

9. A process according to claim 4, wherein the reaction is optionally carried out in the presence of lithium chloride.

10. A process according to any of claims 1 to 9, wherein the molar amount of the chelating ligand is from 1 to 20%, preferably from 1 to 10%.

11. A process according to any of claims 1 to 10, wherein the molar amount of the pre-catalyst Pd complex is from 1 to 5%.

12. A process according to any of claims 1 to 11, wherein the ratio pre-catalyst / ligand is from 0.25 to 1, preferably from 0.3 to 1 and more preferably is about 1.

13. A process according to any of claims 1 to 12, wherein the aryl bromides are chosen from the bromides where the aryl is a substituted or unubstituted 5 to 7 members single aromatic or heteroaromatic radical comprising 0 to 4 heteroatoms chosen from oxygen, sulphur or nitrogen or a bi or tricyclic substituted or unsubstituted aromatic or heteroaromatic radical comprising 6 to 14 members and 0 to 4 heteroatoms chosen from oxygen, sulphur or nitrogen and which can be saturated or partially saturated on the cycles that are not bromo-substituted.

14. A process according to any of claims 1 to 12, wherein the vinyl bromides can be chosen from the substituted or unsubstituted bromides where the vinyl radical is a vinyl derivative where the bromo is substituted on an unsaturated carbon and where the radical can be an aliphatic linear or branched radical having from 2 to 10 carbon atoms and that comprise heteroatoms chosen from oxygen, sulphur or nitrogen, or a mono, bi or tri cyclic radical comprising from 5 to 14 members, that can be carbocyclic or heterocyclic comprising 0 to 4 heteroatoms chosen from oxygen, sulphur or nitrogen and which can be partially saturated and where the bromo is substituted on an unsaturated carbon.

15. A process according to claim 13 or 14, wherein the substituants are chosen from halogen atoms, or radicals chosen from alkyl, halogenoalkyl or polyhalogenoalkyl, alkoxy, acyl, alkoxycarbonyl, alkylaminoalkyl, dialkylaminoalkyl, alkoxycarbonylaminoalkyl or phenyl, the alkyl portions or radicals and the alkyl portion in the acyl radical being linear or branched and having 1 to 10 carbon atoms.

16. A process according to claim 13, wherein the aryl radicals are chosen from phenyl or naphtyl, that can be substituted with halogen atoms chosen from chloro, fluoro, bromo or iodo, or substituted with radicals chosen from linear or branched alkyl or alkoxy having 1 to 4 carbon atoms, trifluoromethyl, acyl or alkoxycarbonyl where the alkyl portions are linear or branched and contain 1 to 4 carbon atoms, dialkylaminoalkyl, methoxycarbonylaminoalkyl, ethoxycarbonyl-aminoalkyl or t.butoxycarbonylaminoalkyl or phenyl.

17. A process according to claim 14, wherein the vinyl radicals are chosen from vinyl or indene-2-yl, that can be substituted with halogen atoms chosen from chloro, fluoro, bromo or iodo, or substituted with radicals chosen from linear or branched alkyl or alkoxy having 1 to 4 carbon atoms, trifluoromethyl, acyl, alkoxycarbonyl where the alkyl portions are linear or branched and contain 1 to 4 carbon atoms, dialkylaminoalkyl, methoxycarbonylaminoalkyl, ethoxycarbonyl-aminoalkyl or t.butoxycarbonylaminoalkyl or phenyl.

18. A process according to any of claims 1 to 17, wherein the concentration of the vinyl or aryl bromide is from 0.01 to 1 M, preferably from 0.1 to 0.8 M and more preferably from 0.1 and 0.4 M.

19. A process according any of claims 1 to 13, 15 and 16, wherein *N-tert-*butoxycarbonyl-*N*-methyl-4-bromobenzylamine is reacted with a carbon monoxide source comprising lithium formate and acetic acid anhydride, in the presence of diisopropyl ethyl amine and in the presence of the combination of palladium complexe Pd₂(dba)₃ with the chelating ligand dppf (1,1-bis[diphenyl-phosphino]ferrocene) in the ratio palladium pre-catalyst/ligand of 5/20, at a temperature of 120°C, in dimethylformamide, in the presence of lithium chloride.

20. A process according any of claims 1 to 13, 15 and 16, wherein *N-tert-*butoxycarbonyl-*N*-methyl-4-bromobenzylamine is reacted with a carbon monoxide source comprising lithium formate and acetic acid anhydride, in the presence of diisopropyl ethyl amine and in the presence of the combination of palladium complexe Pd(OAc)₂ with the chelating ligand dppf (1,1-bis[diphenyl-phosphino]ferrocene) in the ratio palladium pre-catalyst/ligand of 5/5, at a temperature of 120°C, in N-methyl-pyrrolidone.

21. A process according any of claims 1 to 13, 15 and 16, wherein *N-tert-*butoxycarbonyl-*N*-methyl-4-bromobenzylamine is reacted with a carbon monoxide source comprising lithium formate and acetic acid anhydride, in the presence of diisopropyl ethyl amine and in the presence of the combination of palladium complexe Pd(OAc)₂ with the chelating ligand dppf (1,1-bis[diphenyl-phosphino]ferrocene) in the ratio palladium pre-catalyst/ligand of 2.5/2.5, at a temperature of 120°C, in dimethylformamide.

22. A process according any of claims 1 to 13, 15 and 16, wherein bromotoluene is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 1/1 at a temperature of 120°C in dimethylformamide.

23. A process according any of claims 1 to 13, 15 and 16, wherein 4-bromofluorobenzol is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 1/1 at a temperature of 120°C in dimethylformamide.

24. A process according any of claims 1 to 13, 15 and 16, wherein 4-anisole bromide is reacted with a carbon monoxide source comprising lithium formate and acetic acid anhydride, in the presence of diisopropyl ethyl amine and in the presence of the combination of palladium complexe Pd(OAc)₂ with the chelating ligand dppf (1,1-bis[diphenylphosphino]ferrocene) in the ratio palladium pre-catalyst/ligand of 1/1 at a temperature of 120°C in dimethylformamide.

25. A process according any of claims 1 to 13, 15 and 16, wherein 2-bromoindene is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 1/1 at a temperature of 120°C in dimethylformamide.

26. A process according any of claims 1 to 12, 14, 15 and 17, wherein 1,4-dibromobenzol is reacted with a carbon monoxide source comprising lithium formate and acetic acid anhydride, in the presence of diisopropyl ethyl amine and in the presence of the combination of palladium complexe Pd(OAc)₂ with the chelating ligand dppf(1,1-bis[diphenylphosphino]ferrocene) in the ratio palladium pre-catalyst/ligand of 1/1 at a temperature of 120°C in dimethylformamide.

## Patentansprüche

1. Verfahren zur Herstellung von Aryl- oder Vinylcarbonsäuren durch Pd-katalysierte Hydroxycarbonylierung von Aryl- und Vinylbromiden, wobei die Aryl- oder Vinylbromide mit einer Kohlenmonoxidquelle, die Lithiumformiat und ein Carbonsäureanhydrid umfasst, in Gegenwart der Kombination von Palladiumkomplexen mit dem chelatbildenden Liganden dppf (1,1-Bis-[diphenylphosphino]ferrocen) umgesetzt werden.

2. Verfahren nach Anspruch 1, wobei der Pd-Katalysator aus den Kombinationen von Palladiumkomplexen (Vorkatalysator): PdCl₂, Pd₂(dba)₃ oder Pd(OAc)₂ mit dem chelatbildenden Liganden dppf ausgewählt ist.

3. Verfahren nach Anspruch 1 oder 2, wobei das Carbonsäureanhydrid Essigsäureanhydrid ist.

4. Verfahren nach einem der Ansprüche 1 bis 3, wobei die Reaktion in Gegenwart eines tertiären Amins in einem polaren Lösungsmittel bei einer Temperatur zwischen 60 und 200°C durchgeführt wird.

5. Verfahren nach Anspruch 4, wobei das tertiäre Amin ausgewählt ist aus Triethylamin, Diisopropylethylamin, Tetramethylethylendiamin, Tributylamin, Pyridin, N-Methylmorpholin, Tetramethylharnstoff, Methylpyrrolidinon, 4-Dimethylaminopyridin, Protonenschwamm oder Dimethylanilin.

6. Verfahren nach Anspruch 4 oder 5, wobei die Reaktion bei einer Temperatur von 80 bis 150°C, vorzugsweise 80 bis 120°C und insbesondere 120°C durchgeführt wird.

7. Verfahren nach einem der Ansprüche 4 bis 6, wobei das polare Lösungsmittel ein Amid ist.

8. Verfahren nach Anspruch 7, wobei das Amid ausgewählt ist aus Dimethylformamid und N-Methyl-2-pyrrolidon.

9. Verfahren nach Anspruch 4, wobei die Reaktion gegebenenfalls in Gegenwart von Lithiumchlorid durchgeführt wird.

10. Verfahren nach einem der Ansprüche 1 bis 9, wobei die molare Menge des chelatbildenden Liganden 1 bis 20 %, vorzugsweise 1 bis 10 % beträgt.

11. Verfahren nach einem der Ansprüche 1 bis 10, wobei die molare Menge des Vorkatalysators Pd-Komplex 1 bis 5 % beträgt.

12. Verfahren nach einem der Ansprüche 1 bis 11, wobei das Verhältnis von Vorkatalysator/Ligand 0,25 bis 1, vorzugsweise 0,3 bis 1 und insbesondere etwa 1 beträgt.

13. Verfahren nach einem der Ansprüche 1 bis 12, wobei die Arylbromide ausgewählt sind aus den Bromiden, wobei das Aryl ein substituierter oder unsubstituierter, 5- bis 7-gliedriger, einzelner, aromatischer oder heteroaromatischer Rest, der 0 bis 4 Heteroatome ausgewählt aus Sauerstoff, Schwefel oder Stickstoff umfasst, oder ein bi- oder tricyclischer, substituierter oder unsubstituierter, aromatischer oder heteroaromatischer Rest ist, der 6- bis 14-gliedrig ist und 0 bis 4 Heteroatome ausgewählt aus Sauerstoff, Schwefel oder Stickstoff umfasst, und in den Ringen, die nicht bromsubstituiert sind, gesättigt oder ungesättigt sein kann.

14. Verfahren nach einem der Ansprüche 1 bis 12, wobei die Vinylbromide ausgewählt sein können aus den substituierten oder unsubstituierten Bromiden, wobei der Vinylrest ein Vinylderivat ist, wobei das Brom an einem ungesättigten Kohlenstoff substituiert ist und wobei der Rest ein aliphatischer linearer oder verzweigter Rest mit 2 bis 10 Kohlenstoffatomen sein kann und Heteroatome ausgewählt aus Sauerstoff, Schwefel oder Stickstoff umfasst, oder ein mono-, bi- oder tricyclischer Rest ist, der 5- bis 14-gliedrig ist, carbocyclisch oder heterocyclisch sein kann und 0 bis 4 Heteroatome ausgewählt aus Sauerstoff, Schwefel oder Stickstoff umfasst, und der teilweise gesättigt sein kann, und wobei das Brom an einem ungesättigten Kohlenstoff substituiert ist.

15. Verfahren nach Anspruch 13 oder 14, wobei die Substituenten ausgewählt sind aus Halogenatomen oder Resten ausgewählt aus Alkyl, Halogenalkyl oder Polyhalogenalkyl, Alkoxy, Acyl, Alkoxycarbonyl, Alkylaminoalkyl, Dialkylaminoalkyl, Alkoxycarbonylaminoalkyl oder Phenyl, wobei die Alkylanteile oder -reste und der Alkylanteil in dem Acylrest linear oder verzweigt sind und 1 bis 10 Kohlenstoffatome aufweisen.

16. Verfahren nach Anspruch 13, wobei die Arylreste ausgewählt sind aus Phenyl oder Naphthyl, das mit Halogenatomen ausgewählt aus Chlor, Fluor, Brom oder Iod substituiert sein kann, oder mit Resten ausgewählt aus linearem oder verzweigtem Alkyl oder Alkoxy mit 1 bis 4 Kohlenstoffatomen, Trifluormethyl, Acyl oder Alkoxycarbonyl, wobei die Alkylanteile linear oder verzweigt sind und 1 bis 4 Kohlenstoffatome enthalten, Dialkylaminoalkyl, Methoxycarbonylaminoalkyl, Ethoxycarbonyl-aminoalkyl oder tert.-Butoxycarbonylaminoalkyl oder Phenyl substituiert sein kann.

17. Verfahren nach Anspruch 14, wobei die Vinylreste ausgewählt sind aus Vinyl oder Inden-2-yl, das mit Halogenatomen ausgewählt aus Chlor, Fluor, Brom oder Iod substituiert sein kann, oder mit Resten ausgewählt aus linearem oder verzweigtem Alkyl oder Alkoxy mit 1 bis 4 Kohlenstoffatomen, Trifluormethyl, Acyl oder Alkoxycarbonyl, wobei die Alkylanteile linear oder verzweigt sind und 1 bis 4 Kohlenstoffatome enthalten, Dialkylaminoalkyl, Methoxycarbonylaminoalkyl, Ethoxycarbonylaminoalkyl oder tert.-Butoxycarbonylaminoalkyl oder Phenyl substituiert sein kann.

18. Verfahren nach einem der Ansprüche 1 bis 17, wobei die Konzentration des Vinyl- oder Arylbromids 0,01 bis 1 M, vorzugsweise 0,1 bis 0,8 M und insbesondere 0,1 bis 0,4 M beträgt.

19. Verfahren nach einem der Ansprüche 1 bis 13, 15 und 16, wobei N-tert.-Butoxycarbonyl-N-methyl-4-brombenzylamin mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(dba)₃ mit dem chelatbildenden Liganden dppf (1,1-Bis-[diphenylphosphino]ferrocen) in dem Verhältnis von Palladiumvorkatalysator/Ligand von 5/20 bei einer Temperatur von 120°C in Dimethylformamid in Gegenwart von Lithiumchlorid umgesetzt wird.

20. Verfahren nach einem der Ansprüche 1 bis 13, 15 und 16, wobei N-tert.-Butoxycarbonyl-N-methyl-4-brombenzylamin mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[di-phenylphosphino]ferrocen) in dem Verhältnis von Palladiumvorkatalysator/Ligand von 5/5 bei einer Temperatur von 120°C in N-Methylpyrrolidon umgesetzt wird.

21. Verfahren nach einem der Ansprüche 1 bis 13, 15 und 16, wobei N-tert.-Butoxycarbonyl-N-methyl-4-brombenzylamin mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[di-phenylphosphino]ferrocen) in dem Verhältnis von Palladiumvorkatalysator/Ligand von 2,5/2,5, bei einer Temperatur von 120°C in N-Dimethylformamid umgesetzt wird.

22. Verfahren nach einem der Ansprüche 1 bis 13, 15 und 16, wobei Bromtoluol mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[diphenylphosphino]ferrocen) in dem Verhältnis von Palladiumvorkatalysator/Ligand von 1/1 bei einer Temperatur von 120°C in N-Dimethylformamid umgesetzt wird.

23. Verfahren nach einem der Ansprüche 1 bis 13, 15 und 16, wobei 4-Bromfluorbenzol mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[diphenylphosphino]ferrocen) in dem Verhältnis von Palladiumvorkatalysator/Ligand von 1/1, bei einer Temperatur von 120°C in N-Dimethylformamid umgesetzt wird.

24. Verfahren nach einem der Ansprüche 1 bis 13, 15 und 16, wobei 4-Anisolbromid mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[diphenylphosphino]ferrocen) in dem Verhältnis von Palladiumvorkatalysator/Ligand von 1/1 bei einer Temperatur von 120°C in N-Dimethylformamid umgesetzt wird.

25. Verfahren nach einem der Ansprüche 1 bis 13, 15 und 16, wobei 2-Brominden mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[diphenylphosphino]ferrocen) in dem Verhältnis von Palladiumvorkatalysator/Ligand von 1/1 bei einer Temperatur von 120°C in N-Dimethylformamid umgesetzt wird.

26. Verfahren nach einem der Ansprüche 1 bis 12, 14, 15 und 17, wobei 1,4-Dibrombenzol mit einer Kohlenmonoxidquelle, die Lithiumformiat und Essigsäureanhydrid umfasst, in Gegenwart von Diisopropylethylamin und in Gegenwart der Kombination von Palladiumkomplex Pd₂(OAc)₂ mit dem chelatbildenden Liganden dppf (1,1-Bis[diphenylphosphino]ferrocen) in dem Verhältnis von Palladiumvorkatalysator/Ligand von 1/1 bei einer Temperatur von 120°C in N-Dimethylformamid umgesetzt wird.

## Revendications

1. Procédé de préparation d'acides aryle ou vinylcarboxyliques par le biais d'une hydroxycarbonylation de bromures d'aryle et de vinyle catalysée par Pd, dans lequel les bromures d'aryle ou de vinyle sont mis à réagir avec une source de monoxyde de carbone comprenant du formiate de lithium et un anhydride d'acide carboxylique, en présence de la combinaison de complexes de palladium avec le ligand chélatant dppf (1,1-bis[diphénylphosphino]ferrocène).

2. Procédé selon la revendication 1, dans lequel le catalyseur Pd est choisi à partir des combinaisons de complexes de palladium (pré-catalyseur) : PdCl₂, Pd₂(dba)₃ ou Pd(OAc)₂ avec le ligand chélatant dppf.

3. Procédé selon la revendication 1 ou 2, dans lequel l'anhydride d'acide carboxylique est un anhydride d'acide acétique.

4. Procédé selon l'une quelconque des revendications 1 à 3, dans lequel la réaction est réalisée en présence d'une amine tertiaire, dans un solvant polaire à une température comprise entre 60 et 200 °C.

5. Procédé selon la revendication 4, dans lequel l'amine tertiaire est choisie parmi de la triéthylamine, de la diisopropyléthylamine, de la tétraméthyléthylènediamine, de la tributylamine, de la pyridine, de la N-méthylmorpholine, de la tétraméthylurée, de la méthylpyrrolidinone, de la 4-diméthylaminopyridine, une éponge à protons ou de la diméthylaniline.

6. Procédé selon la revendication 4 ou 5, dans lequel la réaction est réalisée à une température de 80 à 150 °C, de préférence 80 à 120 °C et plus préférablement, 120 °C.

7. Procédé selon l'une quelconque des revendications 4 à 6, dans lequel le solvant polaire est un amide.

8. Procédé selon la revendication 7, dans lequel l'amide est choisi parmi du diméthylformamide et de la N-méthyl-2-pyrrolidone.

9. Procédé selon la revendication 4, dans lequel la réaction est éventuellement réalisée en présence de chlorure de lithium.

10. Procédé selon l'une quelconque des revendications 1 à 9, dans lequel la quantité molaire du ligand chélatant est de 1 à 20 %, de préférence de 1 à 10 %.

11. Procédé selon l'une quelconque des revendications 1 à 10, dans lequel la quantité molaire du complexe pré-catalyseur Pd est de 1 à 5 %.

12. Procédé selon l'une quelconque des revendications 1 à 11, dans lequel le rapport pré-catalyseur/ligand est de 0,25 à 1, de préférence de 0,3 à 1, et plus préférablement, ce rapport est d'environ 1.

13. Procédé selon l'une quelconque des revendications 1 à 12, dans lequel les bromures d'aryle sont choisis parmi les bromures où l'aryle est un radical aromatique ou hétéroaromatique unique à 5 à 7 chaînons substitué ou non-substitué comprenant de 0 à 4 hétéroatomes choisis parmi l'oxygène, le soufre ou l'azote ou un radical aromatique ou hétéroaromatique bi- ou tricyclique substitué ou non-substitué comprenant de 6 à 14 chaînons et de 0 à 4 hétéroatomes choisis parmi l'oxygène, le soufre ou l'azote et qui peut être saturé ou partiellement saturé sur les cycles qui ne sont pas à substitution bromo.

14. Procédé selon l'une quelconque des revendications 1 à 12, dans lequel les bromures de vinyle peuvent être choisis parmi les bromures substitués ou non-substitués où le radical de vinyle est un dérivé de vinyle où le brome est substitué sur un carbone non-saturé et où le radical peut être un radical linéaire ou ramifié aliphatique ayant de 2 à 10 atomes de carbone et qui comprennent des hétéroatomes choisi parmi l'oxygène, le soufre ou l'azote, ou un radical mono-, bi- ou tricyclique comprenant de 5 à 14 chaînons, qui peuvent être carbocycliques ou hétérocycliques comprenant de 0 à 4 hétéroatomes choisis parmi l'oxygène, le soufre ou l'azote et qui peuvent être partiellement saturés et où le brome est substitué sur un carbone non-saturé.

15. Procédé selon la revendication 13 ou 14, dans lequel les substituants sont choisis parmi des atomes d'halogène, ou des radicaux choisis parmi alkyle, halogénoalkyle ou polyhalogénoalkyle, alcoxy, acyle, alcoxycarbonyle, alkylaminoalkyle, dialkylaminoalkyle, alcoxycarbonylaminoalkyle ou phényle, les parties ou radicaux alkyle et la partie alkyle dans le radical acyle étant linéaires ou ramifiés et ayant de 1 à 10 atomes de carbone.

16. Procédé selon la revendication 13, dans lequel les radicaux aryle sont choisis parmi du phényle ou du naphtyle, qui peuvent être substitués avec des atomes d'halogène choisis parmi le chlore, le fluor, le brome ou l'iode, ou substitués avec des radicaux choisis parmi alkyle ou alcoxy linéaire ou ramifié ayant de 1 à 4 atomes de carbone, un radical trifluorométhyle, acyle ou alcoxycarbonyle où les parties alkyle sont linéaires ou ramifiées et contiennent de 1 à 4 atomes de carbone, dialkylaminoalkyle, méthoxycarbonylaminoalkyle, éthoxycarbonylaminoalkyle ou t.butoxycarbonylaminoalkyle ou phényle.

17. Procédé selon la revendication 14, dans lequel les radicaux de vinyle sont choisis parmi du vinyle ou de l'indène-2-yle, qui peuvent être substitués avec des atomes d'halogène choisis parmi le chlore, le fluor, le brome ou l'iode, ou substitués avec des radicaux choisis parmi alkyle ou alcoxy linéaire ou ramifié ayant de 1 à 4 atomes de carbone, un radical trifluorométhyle, acyle, alcoxycarbonyle où les parties alkyle sont linéaires ou ramifiées et contiennent de 1 à 4 atomes de carbone, dialkylaminoalkyle, méthoxycarbonylaminoalkyle, éthoxycarbonylaminoalkyle ou t.butoxycarbonylaminoalkyle ou phényle.

18. Procédé selon l'une quelconque des revendications 1 à 17, dans lequel la concentration en bromure de vinyle ou d'aryle est de 0,01 à 1 M, de préférence de 0,1 à 0, 8 M et plus préférablement de 0,1 à 0,4 M.

19. Procédé selon l'une quelconque des revendications 1 à 13, 15 et 16, dans lequel la N-tert-butoxycarbonyl-*N*-méthyl-4-bromobenzylamine est mise à réagir avec une source de monoxyde de carbone comprenant du formiate de lithium et un anhydride d'acide acétique, en présence d'une diisopropyléthylamine et en présence de la combinaison d'un complexe de palladium Pd₂(dba)₃ avec le ligand chélatant dppf(1,1-bis[diphénylphosphino]ferrocène) selon le rapport de pré-catalyseur de palladium/ligand de 5/20, à une température de 120 °C, dans du diméthylformamide, en présence de chlorure de lithium.

20. Procédé selon l'une quelconque des revendications 1 à 13, 15 et 16, dans lequel de la *N*-tert-butoxycarbonyl-*N*-méthyl-4-bromobenzylamine est mise à réagir avec une source de monoxyde de carbone comprenant du formiate de lithium et un anhydride d'acide acétique, en présence de diisopropyléthylamine et en présence de la combinaison d'un complexe de palladium complexe Pd(OAc)₂ avec le ligand chélatant dppf (1,1-bis[diphényl-phosphino]ferrocène) selon le rapport de pré-catalyseur de palladium/ligand de 5/5, à une température de 120 °C, dans de la N-méthyl-pyrrolidone.

21. Procédé selon l'une quelconque des revendications 1 à 13, 15 et 16, dans lequel de la *N*-tert-butoxycarbonyl-*N*-méthyl-4-bromobenzylamine est mise à réagir avec une source de monoxyde de carbone comprenant du formiate de lithium et un anhydride d'acide acétique, en présence de diisopropyléthylamine et en présence de la combinaison d'un complexe de palladium Pd(OAc)₂ avec le ligand chélatant dppf(1,1-bis[diphényl-phosphino]ferrocène) selon le rapport de pré-catalyseur de palladium/ligand de 2,5/2,5, à une température de 120 °C, dans du diméthylformamide.

22. Procédé selon l'une quelconque des revendications 1 à 13, 15 et 16, dans lequel du bromotoluène est mis à réagir avec une source de monoxyde de carbone comprenant du formiate de lithium et un anhydride d'acide acétique, en présence de diisopropyléthylamine et en présence de la combinaison d'un complexe de palladium Pd(OAc)₂ avec le ligand chélatant dppf(1,1-bis[diphénylphosphino]ferrocène) selon le rapport de pré-catalyseur de palladium/ligand de 1/1 à une température de 120 °C dans du diméthylformamide.

23. Procédé selon l'une quelconque des revendications 1 à 13, 15 et 16, dans lequel du 4-bromofluorobenzol est mis à réagir avec une source de monoxyde de carbone comprenant du formiate de lithium et un anhydride d'acide acétique, en présence de diisopropyléthylamine et en présence de la combinaison d'un complexe de palladium Pd(OAc)₂ avec le ligand chélatant dppf(1,1-bis[diphénylphosphino]ferrocène) selon le rapport de pré-catalyseur de palladium/ligand de 1/1 à une température de 120 °C dans du diméthylformamide.

24. Procédé selon l'une quelconque des revendications 1 à 13, 15 et 16, dans lequel du bromure de 4-anisole est mis à réagir avec une source de monoxyde de carbone comprenant du formiate de lithium et un anhydride d'acide acétique, en présence de diisopropyléthylamine et en présence de la combinaison d'un complexe de palladium Pd(OAc)₂ avec le ligand chélatant dppf(1,1-bis[diphénylphosphino]ferrocène) selon le rapport de pré-catalyseur de palladium/ligand de 1/1 à une température de 120 °C dans du diméthylformamide.

25. Procédé selon l'une quelconque des revendications 1 à 13, 15 et 16, dans lequel du 2- bromoindène est mis à réagir avec une source de monoxyde de carbone comprenant du formiate de lithium et un anhydride d'acide acétique, en présence de diisopropyléthylamine et en présence de la combinaison d'un complexe de palladium Pd(OAc)₂ avec le ligand chélatant dppf(1,1-bis[diphénylphosphino]ferrocène) selon le rapport de pré-catalyseur de palladium/ligand de 1/1 à une température de 120 °C dans du diméthylformamide.

26. Procédé selon l'une quelconque des revendications 1 à 12, 14, 15 et 17, dans lequel du 1,4-dibromobenzol est mis à réagir avec une source de monoxyde de carbone comprenant du formiate de lithium et un anhydride d'acide acétique, en présence de diisopropyléthylamine et en présence de la combinaison d'un complexe de palladium Pd(OAc)₂ avec le ligand chélatant dppf(1,1-bis[diphénylphosphino]ferrocène) selon le rapport de pré-catalyseur de palladium/ligand de 1/1 à une température de 120 °C dans du diméthylformamide.
